(19) Europäisches Patentamt
European Patent Office
Office européen des brevets

(11) Publication number: **0 601 520 A1**

(12) # EUROPEAN PATENT APPLICATION

(21) Application number: **93119638.0**

(51) Int. Cl.[5]: **C07H 19/073**, A61K 31/70

(22) Date of filing: **06.12.93**

(30) Priority: **07.12.92 JP 326332/92**
**21.07.93 JP 179716/93**

(43) Date of publication of application:
**15.06.94 Bulletin 94/24**

(84) Designated Contracting States:
**BE CH DE ES FR GB IT LI NL SE**

(71) Applicant: **MITSUI TOATSU CHEMICALS, Inc.**
**2-5 Kasumigaseki 3-chome**
**Chiyoda-Ku Tokyo 100(JP)**

(72) Inventor: **Fukazawa, Nobuyuki**
**2-25-17 Tobudai**
**Mobara-shi, Chiba-ken 297(JP)**
Inventor: **Kawauchi, Nobuya, 401 Mitsui Toatsu**
**Higashitotsuka-Ryo,**
**3-42-7, Hirado,**
**Totsuka-ku**
**Yokohama-shi, Kanagawa-ken 244(JP)**
Inventor: **Ishibashi, Hiroki**
**Miyanodai-Ryo 2142 Tougou**
**Mobara-shi, Chiba-ken 297(JP)**
Inventor: **Shimada, Shizuo**
**252-13 Naganuma-chou,**
**Inage-ku**
**Chiba-shi, Chiba-ken 263(JP)**
Inventor: **Iwata, Daiji**
**Fujimi Dai 2 Aparto 48,**
**2225-1 Tougou**
**Mobara-shi, Chiba-ken 297(JP)**
Inventor: **Takebayashi, Nobuo**
**Tounodai-Shataku 251,**
**90-1 Machibo**
**Mobara-shi, Chiba-ken 297(JP)**
Inventor: **Edatsugi, Hajime**
**Miyanodai-Ryo 2142 Tougou**
**Mobara-shi, Chiba-ken 297(JP)**
Inventor: **Sobajima, Takeshi**
**Miyanodai-Ryo 2142 Tougou**
**Mobara-shi, Chiba-ken 297(JP)**
Inventor: **Yano, Osamu**
**601 Ochiai**
**Hadano-shi, Kanagawa-ken 257(JP)**

(74) Representative: **VOSSIUS & PARTNER**
**Siebertstrasse 4**
**D-81675 München (DE)**

(54) **Trifluorothymidine derivatives, process for producing the same and anti-cancer agent containing the same.**

(57) A thioester derivative of 3'-O-propargyltrifluorothymidine, which is useful as an anti-cancer agent is disclosed. The thioester derivative according to the present invention is represented by the formula (1):

EP 0 601 520 A1

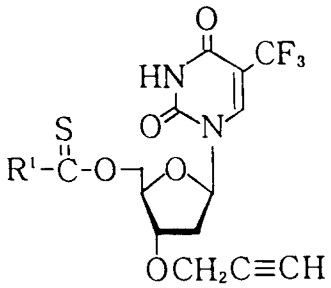

(1)

(wherein $R^1$ represents phenyl group, phenyl group substituted with 1 to 5 halogen atoms, phenyl group substituted with 1 to 5 $C_1$ - $C_4$ alkyl groups, phenyl group substituted with 1 to 5 $C_1$ - $C_4$ alkoxyl groups, phenyl group substituted with 1 to 5 $C_1$ - $C_4$ alkylthio groups, phenyl group substituted with 1 to 5 $C_1$ - $C_5$ alkanoylamino groups, phenyl group substituted with 1 to 5 trifluoromethyl groups, furyl group, thienyl group or pyrrolyl group).

This invention relates to a novel trifluorothymidine derivative having anti-cancer property.

Trifluorothymidine (hereinafter also referred to as "F3Thd") is a nucleic acid metabolism antagonist first synthesized by Heidelberger et al (Journal of the American Chemical Society Vol. 84, p.3597, 1962). The pharmacological properties of this compound has been studied. It is known that this compound has anti-viral activity rather than anti-cancer activity and is effective against infectious diseases caused by, for example, herpes virus and vaccinia virus. As for anti-tumor activity, it has been reported that this compound exhibits excellent anti-tumor activity against experimental model tumors such as L1210 leukemia and adenocarcinoma-755 (Cancer Research, Vol. 24, p.1979, 1964). However, since this compound is not well absorbed when administered orally and is rapidly metabolized in the body, in the clinical applications, the anti-tumor activity of this compound is not so high as that shown in in vitro studies using the model tumors. Further, this compound has side effects such as inhibition of bone marrow.

Thus, a novel F3Thd derivative is desired, which has higher anti-tumor activity in vivo and which has less side effects than F3Thd.

F3Thd derivatives having anti-tumor activities are disclosed in Japanese Laid-open patent application (Kokai) Nos. 58-152898, 59-36696, 60-56996 and 62-187482 and Chemical Pharmaceutical Bulletin Vol. 37, p.2287, 1989. However, the above-mentioned desire is not well satisfied with these F3Thd derivatives. Particularly, these known F3Thd derivatives are rapidly metabolized (conjugation and the like) and excreted, so that the pharmacological effect given by the active moiety F3Thd is not sustained for a long time. Thus, no F3Thd derivatives which are useful in clinical applications are known.

Accordingly, an object of the present invention is to provide a novel F3Thd derivative which is more absorbed when administered orally and less metabolized (conjugation and the like) and less excreted than F3Thd and the known derivatives thereof, so that the kinetics of the drug and its active moiety in the blood are improved and so the anti-tumor activity in vivo is promoted, and which has less side effects than the conventional F3Thd and derivatives thereof.

Another object of the present invention is to provide an anti-cancer agent containing the novel F3Thd derivative of the present invention as an effective ingredient.

Still another object of the present invention is to provide processes for producing the novel F3Thd derivatives according to the present invention.

In order to develop a compound which is more absorbed when orally administered and less metabolized and less excreted and which has less side effects than F3Thd and known derivatives thereof, the present inventors tried to improve the kinetics of the compound in the blood while keeping the anti-tumor activity of F3Thd per se. More particularly, the present inventors tried to develop a compound which reversibly releases F3Thd in vivo, that is, a prodrug of F3Thd. For this, the present inventors introduced various substituents on 3'- and 5'-positions of the deoxyribose in F3Thd. As a result, it was surprisingly discovered that a group of novel F3Thd derivatives in which 3'-position is substituted with propargyl group show much higher absorption when administered orally and much higher anti-tumor activities than the known derivatives (co-pending U.S. patent application No. 07/894,608 and EP 0 517 262 A1). This is presumably because that the propargyl group is slowly liberated by an enzyme and so the active moiety F3Thd is slowly released for a long time, so that the rates of metabolism such as conjugation and excretion are slowed accordingly. Further, since the F3Thd derivatives of the present invention slowly release F3Thd, the side effects such as damages to alimentary canal are largely reduced.

These objects, i.e. the improvement of trifluorothymidine derivatives in order to reduce the conjugation and excretion, thereby retaining the slow release of trifluorothymidine in vivo, have been achieved by the finding that 3'-O-propargyltrifluorothymidine derivatives having a substituent group containing a sulfur atom on the 5'-position are slowly decomposed chemically and enzymatically so that continuous and sustaining release of trifluorothymidine is attained, and have much higher anti-cancer activities. In the interim, thioester derivatives of 3'-O-propargyltrifluorothymidine attain continuous and sustaining release of trifluorothymidine in vivo, which cannot be expected from their chemical instability, and are well absorbed through oral administration. Further, as a result of anti-cancer activity test using tumor-carrying mice, it was confirmed that the thioester derivatives have much higher anti-cancer activities and lower side effects than 5-fluorouracil derivatives which are widely used clinically at present. Based on the above-described discoveries, the present invention was completed, which provides a novel anti-cancer agent that is demanded.

That is, the present invention provides a thioester derivative of 3'-O-propargyl-trifluorothymidine, which is represented by the formula (1):

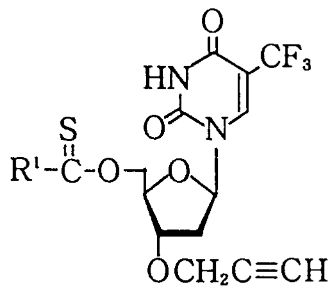

(1)

(wherein $R^1$ represents phenyl group, phenyl group substituted with 1 to 5 halogen atoms, phenyl group substituted with 1 to 5 $C_1$ - $C_4$ alkyl groups, phenyl group substituted with 1 to 5 $C_1$ - $C_4$ alkoxyl groups, phenyl group substituted with 1 to 5 $C_1$ - $C_4$ alkylthio groups, phenyl group substituted with 1 to 5 $C_1$ - $C_5$ alkanoylamino groups, phenyl group substituted with 1 to 5 trifluoromethyl groups, furyl group, thienyl group or pyrrolyl group).

The present invention also provides a process for producing the thioester derivative according to the present invention, comprising reacting a 3'-O-propargyltrifluorothymidine derivative of the formula (3):

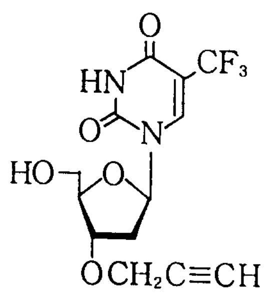

(3)

with a compound of the formula (4):

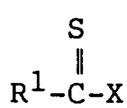

(4)

(wherein $R^1$ represents the same meaning as in formula (1), and X represents a halogen atom) in the presence of a base.

The present invention further provides a process for producing the thioester derivative according to the present invention, comprising reacting a 3'-O-propargyltrifluorothymidine derivative of the formula (3):

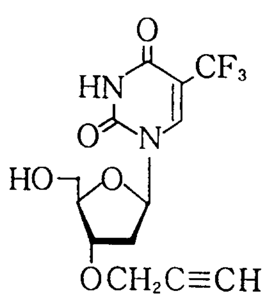

(3)

with a carboxymethyldithioester derivative of the formula (5):

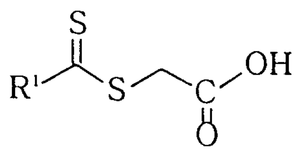

(5)

(wherein $R^1$ represents the same meanings as in formula (1))
in the presence of a base.

The present invention still further provides an anti-cancer agent comprising the thioester derivative according to the present invention as an effective ingredient.

The trifluorothymidine derivatives according to the present invention are more absorbed when orally administered, remain in the blood for a longer time, exhibit smaller side effects, and have higher anti-cancer activities than F3Thd. Thus, it is expected that the trifluorothymidine derivatives according to the present invention can be used clinically as excellent anti-cancer agents.

Fig. 1 shows F3Thd level in plasma, which was generated by decomposition of the compounds according to the present invention in rats.

The trifluorothymidine derivatives according to the present invention will now be described in detail.

$R^1$ in the above-described formula (1) represent phenyl group; phenyl group substituted with 1 to 5 halogen atoms; phenyl group substituted with 1 to 5 $C_1$ -$C_4$ alkyl groups; phenyl group substituted with 1 to 5 $C_1$ - $C_4$ alkoxyl groups; phenyl group substituted with 1 to 5 $C_1$ - $C_4$ alkylthio groups; phenyl group substituted with 1 to 5 $C_1$ - $C_5$ alkanoylamino groups; phenyl group substituted with 1 to 5 trifluoromethyl groups; furyl group; thienyl group or pyrrolyl group.

Examples of the $C_1$ - $C_4$ alkyl groups on the phenyl group include methyl, ethyl, n-propyl, isopropyl, n-butyl, isobutyl and t-butyl groups. Examples of the $C_1$ - $C_4$ alkoxyl group on the phenyl group include methoxy, ethoxy, n-propyloxy, isopropyloxy, n-butoxy, isobutoxy and t-butoxy groups. Examples of the $C_1$ - $C_4$ alkylthio groups on the phenyl group include methylthio, ethylthio, n-propylthio, isopropylthio, n-butylthio, isobutylthio and t-butylthio groups. Examples of $C_1$ - $C_5$ alkanoylamino groups on the phenyl group include formylamino, acetylamino, propionylamino, isotubylylamino, pivaloylamino and butylylamino groups.

Examples of the halogen atoms include fluorine, chlorine, bromine and iodine.

The number of substituents on the phenyl group of $R^1$ may be 0 - 5, preferably 0 - 3.

Among the trifluorothymidine derivatives represented by the formula (1), those represented by the following formula (2) are especially preferred.

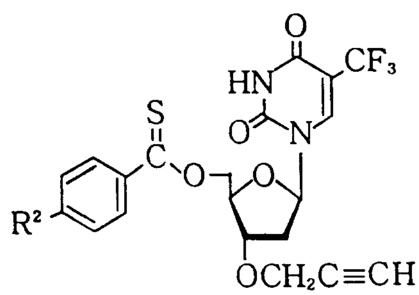

(2)

(wherein $R^2$ represents hydrogen atom, chlorine atom, methyl group or thiomethyl group).

Specific examples of the trifluorothymidine derivatives according to the present invention include
5'-O-thiobenzoyl-3'-O-propargyl-α,α,α-trifluorothymidine,
5'-O-(4-chloro-thiobenzoyl)-3'-O-propargyl-α,α,α-trifluorothymidine,
5'-O-(4-fluoro-thiobenzoyl)-3'-O-propargyl-α,α,α-trifluorothymidine,
5,-O-(4-bromo-thiobenzoyl)-3'-O-propargyl-α,α,α-trifluorothymidine,
5'-O-(4-iodo-thiobenzoyl)-3'-O-propargyl-α,α,α-trifluorothymidine,
5'-O-(2,3-dichloro-thiobenzoyl)-3'-O-propargyl-α,α,α-trifluorothymidine,
5'-O-(2,4-dichloro-thiobenzoyl)-3'-O-propargyl-α,α,α-trifluorothymidine,
5'-O-(2,6-dichloro-thiobenzoyl)-3'-O-propargyl-α,α,α-trifluorothymidine,
5'-O-(3,4-dichloro-thiobenzoyl)-3'-O-propargyl-α,α,α-trifluorothymidine,
5'-O-(2,3,4-trichloro-thiobenzoyl)-3'-O-propargyl-α,α,α-trifluorothymidine,
5'-O-(3,4,5-trichloro-thiobenzoyl)-3'-O-propargyl-α,α,α-trifluorothymidine,

5'-O-(2,3-difluoro-thiobenzoyl)-3'-O-propargyl-α,α,α-trifluorothymidine,
5'-O-(2,4-difluoro-thiobenzoyl)-3'-O-propargyl-α,α,α-trifluorothymidine,
5'-O-(2,6-difluoro-thiobenzoyl)-3'-O-propargyl-α,α,α-trifluorothymidine,
5'-O-(3,4-difluoro-thiobenzoyl)-3'-O-propargyl-α,α,α-trifluorothymidine,
5'-O-(2,3-dibromo-thiobenzoyl)-3'-O-propargyl-α,α,α-trifluorothymidine,
5'-O-(2,4-dibromo-thiobenzoyl)-3'-O-propargyl-α,α,α-trifluorothymidine,
5'-O-(2,6-dibromo-thiobenzoyl)-3'-O-propargyl-α,α,α-trifluorothymidine,
5'-O-(3,4-dibromo-thiobenzoyl)-3'-O-propargyl-α,α,α-trifluorothymidine,
5'-O-(4-methyl-thiobenzoyl)-3'-O-propargyl-α,α,α-trifluorothymidine,
5'-O-(4-ethyl-thiobenzoyl)-3'-O-propargyl-α,α,α-trifluorothymidine,
5'-O-(4-propyl-thiobenzoyl)-3'-O-propargyl-α,α,α-trifluorothymidine,
5'-O-(4-n-butyl-thiobenzoyl)-3'-O-propargyl-α,α,α-trifluorothymidine,
5'-O-(4-t-butyl-thiobenzoyl)-3'-O-propargyl-α,α,α-trifluorothymidine,
5'-O-(2,3-dimethyl-thiobenzoyl)-3'-O-propargyl-α,α,α-trifluorothymidine,
5'-O-(2,4-dimethyl-thiobenzoyl)-3'-O-propargyl-α,α,α-trifluorothymidine,
5'-O-(2,6-dimethyl-thiobenzoyl)-3'-O-propargyl-α,α,α-trifluorothymidine,
5'-O-(3,4-dimethyl-thiobenzoyl)-3'-O-propargyl-α,α,α-trifluorothymidine,
5'-O-(2,3,4-trimethyl-thiobenzoyl)-3'-O-propargyl-α,α,α-trifluorothymidine,
5'-O-(3,4,5-trimethyl-thiobenzoyl)-3'-O-propargyl-α,α,α-trifluorothymidine,
5'-O-(4-methoxy-thiobenzoyl)-3'-O-propargyl-α,α,α-trifluorothymidine,
5'-O-(4-ethoxy-thiobenzoyl)-3'-O-propargyl-α,α,α-trifluorothymidine,
5'-O-(4-propoxy-thiobenzoyl)-3'-O-propargyl-α,α,α-trifluorothymidine,
5'-O-(4-n-butoxy-thiobenzoyl)-3'-O-propargyl-α,α,α-trifluorothymidine,
5'-O-(4-t-butoxy-thiobenzoyl)-3'-O-propargyl-α,α,α-trifluorothymidine,
5'-O-(2,3-dimethoxy-thiobenzoyl)-3'-O-propargyl-α,α,α-trifluorothymidine,
5'-O-(2,4-dimethoxy-thiobenzoyl)-3'-O-propargyl-α,α,α-trifluorothymidine,
5'-O-(2,6-dimethoxy-thiobenzoyl)-3'-O-propargyl-α,α,α-trifluorothymidine,
5,-O-(3,4-dimethoxy-thiobenzoyl)-3'-O-propargyl-α,α,α-trifluorothymidine,
5'-O-(2,3,5-trimethoxy-thiobenzoyl)-3'-O-propargyl-α,α,α-trifluorothymidine,
5'-O-(3,4,5-trimethoxy-thiobenzoyl)-3'-O-propargyl-α,α,α-trifluorothymidine,
5'-O-(4-methylthio-thiobenzoyl)-3'-O-propargyl-α,α,α-trifluorothymidine,
5'-O-(4-ethylthio-thiobenzoyl)-3'-O-propargyl-α,α,α-trifluorothymidine,
5'-O-(4-propylthio-thiobenzoyl)-3'-O-propargyl-α,α,α-trifluorothymidine,
5'-O-(4-n-butylthio-thiobenzoyl)-3'-O-propargyl-α,α,α-trifluorothymidine,
5'-O-(4-t-butylthio-thiobenzoyl)-3'-O-propargyl-α,α,α-trifluorothymidine,
5'-O-(2,3-dimethylthio-thiobenzoyl)-3'-O-propargyl-α,α,α-trifluorothymidine,
5'-O-[2,4-bis(methylthio)-thiobenzoyl]-3'-O-propargyl-α,α,α-trifluorothymidine,
5'-O-[2,6-bis(methylthio)-thiobenzoyl]-3'-O-propargyl-α,α,α-trifluorothymidine,
5'-O-[3,4-bis(methylthio)-thiobenzoyl]-3'-O-propargyl-α,α,α-trifluorothymidine,
3'-O-propargyl-5'-O-(4-acetylaminothiobenzoyl)-α,α,α-trifluorothymidine,
3'-O-propargyl-5'-O-(4-formylaminothiobenzoyl)-α,α,α-trifluorothymidine,
3'-O-propargyl-5'-O-(4-propionylaminothiobenzoyl)-α,α,α-trifluorothymidine,
3'-O-propargyl-5'-O-(4-pivaloylaminothiobenzoyl)-α,α,α-trifluorothymidine,
3'-O-propargyl-5'-O-(4-butylylaminothiobenzoyl)-α,α,α-trifluorothymidine,
3'-O-propargyl-5'-O-(4-isobutylylaminothiobenzoyl)-α,α,α-trifluorothymidine,
3'-O-propargyl-5'-O-(4-trifluoromethyl-thiobenzoyl)-α,α,α-trifluorothymidine,
3'-O-propargyl-5'-O-(2-thiophenecarbothionyl)-α,α,α-trifluorothymidine,
3'-O-propargyl-5'-O-(2-furancarbothionyl)-α,α,α-trifluorothymidine, and
3'-O-propargyl-5'-O-(2-pyrrolecarbothionyl)-α,α,α-trifluorothymidine.

The trifluorothymidine derivatives according to the present invention may be prepared by reacting a 3'-O-propargyl-trifluorothymidine derivative of the formula (3):

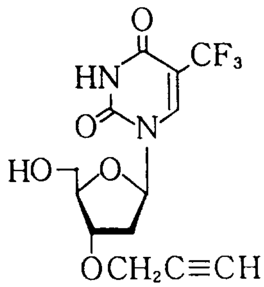

(3)

with a compound of the formula (4):

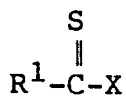

(4)

(wherein $R^1$ represents the same meaning as in the above-described formula (1), and X represents a halogen atom) or with a carboxymethyldithioester derivative of the formula (5):

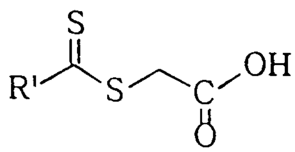

(5)

in the presence of a base.

The trifluorothymidine derivative represented by the formula (3) which is a starting material can be obtained from trifluorothymidine or a trifluorothymidine derivative in which the 5'-position of trifluorothymidine is protected, according to the method described in EP-A-517 262.

Most of the compounds represented by the formula (4), which are also starting materials, are known, and may be produced by, for example, the method described in R. Mayer and S. Scheithauer, Ber., 98 829 (1964).

Most of the compounds represented by the formula (5), which are also starting materials, are known, and may be produced by, for example, the method described in A. Jensen and C. Pedersen, Acta, Chem. Scand. 15, 1087 (1961).

In both reactions, any solvent may be employed as long as it does not influence the reaction. Examples of such a solvent include aprotic solvents such as dichloromethane, chloroform, dioxane, tetrahydrofuran, benzene, toluene, 1,2-dimethoxyethane and dimethylformamide. In both of the reactions employing the compound of the formula (4) and the compound of the formula (5), respectively, tetrahydrofuran is best preferred as the solvent.

Examples of the base which may be employed in both of the reactions include organic bases such as trialkylamines, pyridine, 4-dimethylaminopyridine, picoline, lutidine and imidazole; and inorganic bases such as sodium hydride, potassium hydride, potassium hydroxide, potassium t-butoxide, sodium hydroxide, sodium carbonate and sodium hydrogen carbonate; as well as combinations thereof. Among these bases, in the reaction employing the compound of the formula (4), combination of triethylamine and 4-dimethylaminopyridine is best preferred, and in the reaction employing the compound of the formula (5), combination of sodium hydride and imidazole is best preferred.

In both reactions, the amount of the compound of the formula (4) or (5) may be 1 - 10 times by mole, preferably 1 - 5 times by mole that of the compound of the formula (3), and the amount of the base may be 0.01 - 10 times by mole, preferably 1 - 5 times by mole that of the compound of the formula (3).

In both reactions, the reaction temperature may usually be 0°C (cooling in iced water) to the boiling point of the solvent, preferably 4 - 60°C. The reaction time may usually be 0.5 - 72 hours.

When the compound of the present invention is used as a pharmaceutical for treating a tumor or for

inhibiting a tumor growth, although the dosage of the compound varies depending on the properties of the compound and on the conditions of the patient, the dosage may usually be 50 - 1000 mg/day for an adult in both oral administration and parenteral administration. The above-mentioned dosage may be administered in one time or may be divided into several times.

The compound may be formulated to tablets, granules, powder, suspensions and capsules for oral administration and may be formulated to, for example, suppository for parenteral application. Such pharmaceutical compositions contain effective amount of the compound of the present invention in a pharmaceutically acceptable carrier well-known in the art. For example, for formulating tablets, crystalline cellulose, soft silicic anhydride or the like may be used as an absorbent, and corn starch, lactose, potassium phosphate, magnesium stearate or the like may be used as a vehicle.

As mentioned above, the trifluorothymidine derivatives according to the present invention are effective for treating cancer. Specific examples of the cancer which may be treated by the compound of the present invention may include colorectal cancer, breast cancer, gastric cancer, lung cancer, sarcoma, lymphoma, leukemia and melanoma.

The invention will now be described by way of examples thereof. It should be noted that the examples are presented for the illustration purpose only and should not be interpreted in any restrictive way.

The physical properties of the compounds prepared in the following examples are summarized in Table 1. In Table 1, "R1" means the $R^1$ in the formula (1).

Reference Example 1

5'-O-(t-butyldimethylsilyl)-3'-O-propargyl-α,α,α-trifluorothymidine

(a) A mixture of 197 mg of 5'-O-(t-butyldimethylsilyl)-α,α,α-trifluorothymidine, 110 mg of chloro(t-butyl)-dimethylsilane, 58.9 mg of imidazole and 3.9 ml of dimethylformamide (DMF) was stirred at room temperature for 1 hour. After the reaction, the reaction mixture was concentrated and the resultant was subjected to purification by column chromatography to obtain 5'-O-(t-butyldimethylsilyl)-α,α,α-trifluorothymidine.
Yield: 220 mg

(b) 5'-O-(t-butyldimethylsilyl)-3'-O-propargyl-α,α,α-trifluorothymidine

In 234 ml of tetrahydrofuran (THF), 7.8 g of 5'-O-(t-butyldimethylsilyl)-α,α,α-trifluorothymidine was dissolved and 1.75 g of sodium hydride (purity: 60 wt%) was added to the resulting solution at room temperature. Fifteen minutes later, 3.39 g of propargylbromide was dropped to the mixture and the resultant was allowed to react at room temperature for 5 hours. After the reaction, the reaction mixture was poured into iced water and the resultant was subjected to extraction with ethyl acetate. The extract was concentrated and purified by column chromatography to obtain 5'-O-(t-butyldimethylsilyl)-3'-O-propargyl-α,α,α-trifluorothymidine.
Yield: 7.9 g

Reference Example 2

3'-O-propargyl-α,α,α-trifluorothymidine

In 20 ml of THF, 0.93 g of 3'-O-propargyl-5'-O-(t-butyldimethylsilyl)-α,α,α-trifluorothymidine was dissolved and 2.1 ml of tetra(n-butyl)ammonium fluoride solution in 1 M THF was added to the solution. The resulting mixture was left to stand at room temperature for 11 hours and the reaction mixture was concentrated. The resulting residue was dissolved in ethyl acetate and the solution was washed with iced water. The resultant was extracted with ethyl acetate and the extract was concentrated, followed by purification by column chromatography to obtain 3'-O-propargyl-α,α,α-trifluorothymidine.
Yield: 0.85 g

Reference Example 3

S-(4-trifluoromethyl-thiobenzoyl)thioglycolic acid

In 30 ml of THF, 1.5 g of magnesium metal was suspended and 5 ml of 4-bromobenzotrifluoride was slowly dropped into the suspension at room temperature while vigorously stirring the suspension. The resultant was left to stand at room temperature for 1 hour and the obtained supernatant was dropped into

3.5 ml of carbon disulfide cooled in iced water. The resultant was left to stand overnight at room temperature and the resulting reaction mixture was poured into water. To the obtained aqueous layer, 4.19 g of sodium chloroacetate was added and the resultant was left to stand overnight at room temperature. The resultant was acidified with concentrated hydrochloric acid and the mixture was then extracted with ether. The ether layer was concentrated under reduced pressure and then subjected to crystallization from benzene-hexane to yield 4.1 g of carboxymethyl 4-trifluoromethyldithiobenzoate.

Example 1

3'-O-propargyl-5'-O-(4-methyl-thiobenzoyl)-α,α,α-trifluorothymidine

In 25 ml of THF, 3.3 g of 3'-O-propargyl-α,α,α-trifluorothymidine and 1.35 g of imidazole were dissolved. To this solution, 1.58 g of sodium hydride was added and the resulting mixture was stirred at room temperature for 15 minutes. To the resultant, 2.3 g of S-(4-methyl-thiobenzoyl)thioglycolic acid was added and the resultant was allowed to react at room temperature for 1 hour. The resulting mixture was poured into 5% aqueous ammonium chloride solution and the resultant was shaken, followed by leaving the mixture to stand to attain phase separation. The obtained aqueous layer was subjected to extraction with ethyl acetate and the combined organic layers were washed twice with aqueous saturated sodium chloride solution, followed by drying over anhydrous sodium sulfate. The desiccant was removed by filtration and the filtrate was concentrated under reduced pressure. The obtained residue was subjected to purification by silica gel column chromatography (hexane:ethyl acetate = 2:1 (v/v)) to obtain 3'-O-propargyl-5'-O-(4-methyl-thiobenzoyl)-α,α,α-trifluorothymidine as pale yellow amorphous powder.
Yield: 3.12 g

Example 2

3'-O-propargyl-5'-O-(2,4-dimethoxythiobenzoyl)-α,α,α-trifluorothymidine

In 4 ml of THF, 0.45 g of 3'-O-propargyl-α,α,α-trifluorothymidine and 190 mg of imidazole were dissolved. To this solution, 0.15 g of sodium hydride was added and the resultant was stirred for 15 minutes. To the resulting mixture, 0.37 g of S-(2,4-dimethoxythiobenzoyl)thioglycolic acid was added and the resultant was allowed to react at room temperature for 1 hour. In exactly the same manner as in Example 1, 3'-O-propargyl-5'-O-(2,4-dimethoxythiobenzoyl)-α,α,α-trifluorothymidine was obtained as pale yellow amorphous powder.
Yield: 0.36 g

Example 3

3'-O-propargyl-5'-O-(3,5-dimethyl-thiobenzoyl)-α,α,α-trifluorothymidine

In 4 ml of THF, 0.45 g of 3'-O-propargyl-α,α,α-trifluorothymidine and 190 mg of imidazole were dissolved. To this solution, 0.15 g of sodium hydride was added and the resultant was stirred for 15 minutes. To the resulting mixture, 0.36 g of S-(3,5-dimethylthiobenzoyl)thioglycolic acid was added and the resultant was allowed to react at room temperature for 1 hour. In exactly the same manner as in Example 1, 3'-O-propargyl-5'-O-(3,5-dimethyl-thiobenzoyl)-α,α,α-trifluorothymidine was obtained as pale yellow amorphous powder.
Yield: 0.44 g

Example 4

3'-O-propargyl-5'-O-(4-chlorothiobenzoyl)-α,α,α-trifluorothymidine

In 25 ml of THF, 3.3 g of 3'-O-propargyl-α,α,α-trifluorothymidine and 1.35 g of imidazole were dissolved. To this solution, 1.58 g of sodium hydride was added and the resultant was stirred for 15 minutes. To the resulting mixture, 2.51 g of S-(4-chlorothiobenzoyl)thioglycolic acid was added and the resultant was allowed to react at room temperature for 1 hour. In exactly the same manner as in Example 1, 3'-O-propargyl-5'-O-(4-chlorothiobenzoyl)-α,α,α-trifluorothymidine was obtained as pale yellow amorphous powder.

Yield: 2.93 g

Example 5

3'-O-propargyl-5'-O-(4-fluorothiobenzoyl)-α,α,α-trifluorothymidine

In 25 ml of THF, 3.3 g of 3'-O-propargyl-α,α,α-trifluorothymidine and 1.32 g of imidazole were dissolved. To this solution, 1.38 g of sodium hydride was added and the resultant was stirred for 15 minutes. To the resulting mixture, 2.4 g of S-(4-fluorothiobenzoyl)thioglycolic acid was added and the resultant was allowed to react at room temperature for 1 hour. In exactly the same manner as in Example 1, 3'-O-propargyl-5'-O-(4-fluorothiobenzoyl)-α,α,α-trifluorothymidine was obtained as pale yellow amorphous powder.
Yield: 2.39 g

Example 6

3'-O-propargyl-5'-O-(4-trifluoromethyl-thiobenzoyl)-α,α,α-trifluorothymidine

In 5 ml of THF, 0.33 g of 3'-O-propargyl-α,α,α-trifluorothymidine and 86 mg of imidazole were dissolved. To this solution, 76 mg of sodium hydride was added and the resultant was stirred for 15 minutes. To the resulting mixture, 294 mg of S-(4-trifluoromethylthiobenzoyl)thioglycolic acid was added and the resultant was allowed to react at room temperature for 1 hour. In exactly the same manner as in Example 1, amorphous 3'-O-propargyl-5'-O-(4-trifluoromethyl-thiobenzoyl)-α,α,α-trifluorothymidine was obtained.
Yield: 252 mg

Example 7

3,-O-propargyl-5'-O-(4-methoxythiobenzoyl)-α,α,α-trifluorothymidine

In 5 ml of THF, 0.4 g of 3'-O-propargyl-α,α,α-trifluorothymidine and 104 mg of imidazole were dissolved. To this solution, 92 mg of sodium hydride was added and the resultant was stirred for 15 minutes. To the resulting mixture, 304 mg of S-(4-methoxythiobenzoyl)thioglycolic acid was added and the resultant was allowed to react at room temperature for 1 hour. In exactly the same manner as in Example 1, amorphous 3'-O-propargyl-5'-O-(4-methoxythiobenzoyl)-α,α,α-trifluorothymidine was obtained.
Yield: 364 mg

Example 8

3'-O-propargyl-5'-O-(4-acetylaminothiobenzoyl)-α,α,α-trifluorothymidine

In 6 ml of THF, 410 mg of 3'-O-propargyl-α,α,α-trifluorothymidine and 165 mg of imidazole were dissolved. To this solution, 144 mg of sodium hydride was added and the resultant was stirred for 15 minutes. To the resulting mixture, 365 mg of S-(4-acetylaminobenzoyl)thioglycolic acid was added and the resultant was allowed to react at room temperature for 1 hour. In exactly the same manner as in Example 1, 3'-O-propargyl-5'-O-(4-acetylaminothiobenzoyl)-α,α,α-trifluorothymidine was obtained as pale yellow amorphous powder.
Yield: 334 mg

Example 9

3'-O-propargyl-5'-O-(4-thiomethylthiobenzoyl)-α,α,α-trifluorothymidine

In 95 ml of THF, 5.4 g of 3'-O-propargyl-α,α,α-trifluorothymidine and 2.32 g of imidazole were dissolved. To this solution, 2.0 g of sodium hydride was added and the resultant was stirred for 15 minutes. To the resulting mixture, 4.6 g of S-(4-thiomethylthiobenzoyl)thioglycolic acid was added and the resultant was allowed to react at room temperature for 1 hour. In exactly the same manner as in Example 1, 3'-O-propargyl-5'-O-(4-thiomethylthiobenzoyl)-α,α,α-trifluorothymidine was obtained as pale yellow amorphous powder.

Yield: 4.72 g

Example 10

3'-O-propargyl-5'-O-(4-t-butyl-thiobenzoyl)-α,α,α-trifluorothymidine

In 6 ml of THF, 400 mg of 3'-O-propargyl-α,α,α-trifluorothymidine and 165 mg of imidazole were dissolved. To this solution, 144 mg of sodium hydride was added and the resultant was stirred for 15 minutes. To the resulting mixture, 350 mg of S-(4-t-butylthiobenzoyl)thioglycolic acid was added and the resultant was allowed to react at room temperature for 1 hour. In exactly the same manner as in Example 1, amorphous 3'-O-propargyl-5'-O-(4-t-butyl-thiobenzoyl)-α,α,α-trifluorothymidine was obtained.
Yield: 242 mg

Example 11

3'-O-propargyl-5'-O-(2-thiophenecarbothionyl)-α,α,α-trifluorothymidine

In 5 ml of THF, 500 mg of 3'-O-propargyl-α,α,α-trifluorothymidine and 200 mg of imidazole were dissolved. To this solution, 240 mg of sodium hydride was added and the resultant was stirred for 15 minutes. To the resulting mixture, 342 mg of S-(2-thiophenecarbothionyl)thioglycolic acid was added and the resultant was allowed to react at room temperature for 1 hour. In exactly the same manner as in Example 1, 3'-O-propargyl-5'-O-(2-thiophenecarbothionyl)-α,α,α-trifluorothymidine was obtained as pale yellow amorphous powder.
Yield: 505 mg

Example 12

3'-O-propargyl-5'-O-(2-furancarbothionyl)-α,α,α-trifluorothymidine

In 5 ml of THF, 500 mg of 3'-O-propargyl-α,α,α-trifluorothymidine and 200 mg of imidazole were dissolved. To this solution, 240 mg of sodium hydride was added and the resultant was stirred for 15 minutes. To the resulting mixture, 233 mg of S-(2-furancarbothionyl)thioglycolic acid was added and the resultant was allowed to react at room temperature for 1 hour. In exactly the same manner as in Example 1, 3'-O-propargyl-5'-O-(2-furancarbothionyl)-α,α,α-trifluorothymidine was obtained as pale yellow amorphous powder.
Yield: 356 mg

Example 13

3'-O-propargyl-5'-O-(2-pyrrolecarbothionyl)-α,α,α-trifluorothymidine

In 5 ml of THF, 800 mg of 3'-O-propargyl-α,α,α-trifluorothymidine and 325 mg of imidazole were dissolved. To this solution, 430 mg of sodium hydride was added and the resultant was stirred for 15 minutes. To the resulting mixture, 722 mg of S-(2-pyrrolecarbothionyl)thioglycolic acid was added and the resultant was allowed to react at room temperature for 1 hour. In exactly the same manner as in Example 1, 3'-O-propargyl-5'-O-(2-pyrrolecarbothionyl)-α,α,α-trifluorothymidine was obtained as pale yellow amorphous powder.
Yield: 200 mg

Example 14

3'-O-propargyl-5'-O-thiobenzoyl-α,α,α-trifluorothymidine

In 2 ml of THF, 54 mg of 3'-O-propargyl-α,α,α-trifluorothymidine and 0.1 g of imidazole were dissolved. To this solution, 50 mg of sodium hydride was added and the resultant was stirred for 15 minutes. To the resulting mixture, 64 mg of thiobenzoylthioglycolate was added and the resultant was allowed to react at room temperature for 1 hour. The reaction mixture was poured into 5 ml of aqueous saturated ammonium chloride solution and the resultant was subjected to extraction twice with 10 ml of ethyl acetate. The extract

was dried over anhydrous sodium sulfate and the solvent was evaporated under reduced pressure to obtain pale yellow oil. The thus obtained product was purified by silica gel column chromatography (hexane:ethyl acetate = 1:1 (v/v)) to obtain 3'-O-propargyl-5'-O-thiobenzoyl-α,α,α-trifluorothymidine as yellow oil. The obtained product was crystallized from hexane to obtain 3'-O-propargyl-5'-O-(4-methoxytetrahydropyrane-4-yl)-α,α,α-trifluorothymidine.
Yield: 83 mg

Example 15

3'-O-propargyl-5'-O-thiobenzoyl-α,α,α-trifluorothymidine

In 4 ml of THF, 0.15 g of 3'-O-propargyl-α,α,α-trifluorothymidine, 0.15 ml of triethylamine and 10 mg of N,N-dimethylaminopyridine were dissolved. To this solution, 0.08 ml of thiobenzoyl chloride was added and the resultant was stirred at room temperature for 1 hour. The reaction mixture was poured into 5 ml of iced water and the resultant was subjected to extraction twice with 10 ml of ethyl acetate. The extract was dried over anhydrous sodium sulfate and the solvent was evaporated under reduced pressure to obtain pale yellow oil. The thus obtained product was purified by silica gel column chromatography (hexane:ethyl acetate = 1:1 (v/v)) to obtain 3'-O-propargyl-5'-O-thiobenzoyl-α,α,α-trifluorothymidine as yellow oil. The obtained product was powdered with hexane to obtain 3'-O-propargyl-5'-O-thiobenzoyl-α,α,α-trifluorothymidine.
Yield: 155 mg

Table 1

| Example | R 1 | N M R ; δ p p m | I R c m$^{-1}$ |
|---|---|---|---|
| 1 | 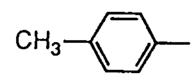 | 2. 18(m, 1H)2. 39(s, 3H)2. 48(s, 1H)2. 76(m, 1H)4. 22(d, 1H) 4. 29(d 1H)4. 51(s, 1H)4. 61(s, 1H)4. 94(s 2H)6. 18(t, 1H) 7. 18(d, 2H)7. 95(s, 1H)7. 98(d, 1H)8. 60(b, 1H) | 3282, 3083 2947, 1701 1605, 1467 1277 |
| 2 | 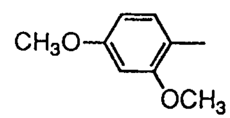 | 2. 28(m, 1H)2. 50(t, 1H)2. 69(m, 1H)3. 78(s, 3H)3. 84(s, 3H) 4. 24(d 1H)4. 29(d, 1H)4. 57(m, 2H)4. 81(dd, 1H)4. 86(dd, 1H) 6. 22(t, 1H)6. 39(d, 1H)6. 50(dd, 1H)7. 90(d, 1H)7. 94(s, 1H) 8. 32(b, 1H) | 3281, 3090 2955, 1729 1701, 1602 1465, 1268 784 |
| 3 | 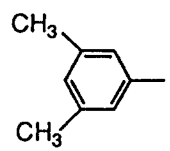 | 2. 21(m, 1H)2. 34(s, 6H)2. 49(t, 1H)2. 79(m, 1H)4. 23(dd, 1H) 4. 30(dd 1H)4. 51(m, 1H)4. 63(m, 1H)4. 88(dd, 1H)9. 95(dd, 1H 6. 17(dd, 2H)7. 20(s, 1H)7. 69(s, 2H)7. 95(s, 1H)8. 64(b, 1H) | 3282, 3080 2921, 1701 1467, 1314 1277, 1199 1036 |
| 4 | 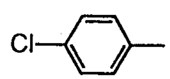 | 2. 21(m, 1H)2. 49(s, 1H)2. 76(m, 1H)4. 23(dd, 1H)4. 30(dd, 1H) 4. 53(m, 2H)4. 89(dd, 1H)4. 95(dd, 1H)6. 17(t, 1H)7. 37(d, 2H) 7. 91(s, 1H)8. 05(dd, 2H)8. 93(s, 1H) | 3283, 3082 2951, 1702 1589, 1467 1277, 1092 |
| 5 | 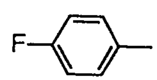 | 2. 21(m, 1H)2. 49(t, 1H)2. 76(m, 1H)4. 23(dd, 1H)4. 30(dd, 1H) 4. 50(m, 1H)4. 59(m, 1H)4. 89(dd, 1H)4. 96(dd, 1H)6. 17(t, 1H) 7. 02(t, 2H)7. 92(s, 1H)8. 14(dd, 2H)8. 91(b, 1H) | 3289, 9081 2951, 1701 1597, 1504 1467, 1276 |

Table 1 (continued)

| Example | R1 | NMR ; δ ppm | IR $cm^{-1}$ |
|---|---|---|---|
| 6 | 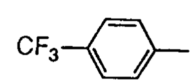 | 8. 63(b, 1H)7. 88(s, 1H)8. 15(d, 2H)7. 65(d, 2H)6. 16(t, 1H) 4. 95(m, 2H)4. 61(m, 1H)4. 53(m, 1H)4. 26(m, 2H)2. 75(m, 1H) 2. 49(m, 1H)2. 18(m, 1H) | 3269, 1702 1467, 1412 1366, 1325 1129, 1109 1069, 851 |
| 7 | 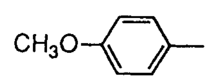 | 9. 26(b, 1H)7. 91(s, 1H)8. 18(d, 2H)6. 85(d, 2H)6. 16(t, 1H) 4. 92(m, 2H)4. 61(m, 1H)4. 52(m, 1H)4. 25(m, 2H)3. 86(s, 3H) 2. 77(m, 1H)2. 50(m, 1H)2. 14(m, 1H) | 3584, 2363 1691, 1598 1467, 1277 1167, 786 |
| 8 | 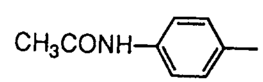 | 9. 21(b, 1H)7. 85(s, 1H)8. 00(d, 2H)7. 56(d, 2H)6. 09(t, 1H) 4. 85(m, 2H)4. 52(m, 1H)4. 44(m, 1H)4. 19(m, 2H)2. 15(s, 3H) 2. 66(m, 1H)2. 44(m, 1H)2. 17(m, 1H) | 3259, 1704 1593, 1533 1469, 1411 1277, 852 760 |
| 9 | 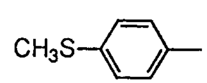 | 9. 49(b, 1H)7. 80(s, 1H)7. 93(d, 2H)7. 10(d, 2H)6. 10(t, 1H) 4. 86(m, 2H)4. 51(m, 1H)4. 43(m, 1H)4. 17(m, 2H)2. 43(s, 3H) 2. 71(m, 1H)2. 41(m, 1H)2. 09(m, 1H) | 3586, 1691 1586, 1467 1403, 1368 1275, 1182 1093, 828 |
| 10 | 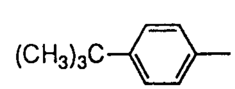 | 9. 60(b, 1H)7. 96(s, 1H)8. 02(d, 2H)7. 39(d, 2H)6. 21(t, 1H) 4. 93(m, 2H)4. 60(m, 1H)4. 51(m, 1H)4. 26(m, 2H)1. 32(s, 9H) 2. 80(m, 1H)2. 50(m, 1H)2. 21(m, 1H) | 2964, 1699 1602, 1465 1368, 1316 1276, 1190 1110, 843 |

Table 1 (continued)

| Example | R 1 | N M R ; δ p p m | I R cm⁻¹ |
|---|---|---|---|
| 11 | 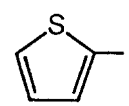 | 2. 24(m, 1H)2. 50(t, 1H)2. 77(m, 1H)4. 22(dd, 1H)4. 30(dd, 1H) 4. 48(m, 1H)4. 59(m, 1H)4. 85(dd, 1H)4. 93(dd, 1H)6. 22(dd, 1H 7. 09(t, 1H)7. 57(d, 1H)7. 92(d, 1H)8. 00(s, 1H)8. 60(b, 1H) | 3449, 1702 1515, 1472 1412, 1276 1200, 1166 1089, 1034 |
| 12 | 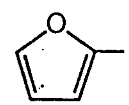 | 2. 32(m, 1H)2. 49(t, 1H)2. 70(m, 1H)4. 21(dd, 1H)4. 29(dd, 1H) 4. 45(m, 1H)4. 61(m, 1H)4. 77(dd, 1H)4. 92(dd, 1H)6. 33(dd, 2H 6. 52(dd, 1H)7. 45(d, 1H)7. 49(d, 1H)8. 16(s, 2H)8. 30(b, 1H) | 3448, 1702 1473, 1245 1211, 1166 1033, 954 758 |
| 13 | 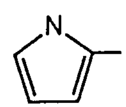 | 2. 22(m, 1H)2. 49(t, 1H)2. 77(m, 1H)4. 21(dd, 1H)4. 29(dd, 1H) 4. 51(m, 1H)4. 57(m, 1H)4. 82(dd, 1H)4. 96(dd, 1H)6. 17(dd, 1H 6. 28(dd, 1H)6. 73(b, 1H)7. 09(b, 1H)8. 32(b, 1H)9. 49(b, 1H) | 3328, 1693 1540, 1485 1397, 1341 1283, 1206 1132, 759 |
| 14 | 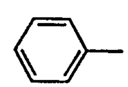 | 2. 19(m, 1H)2. 48(m, 1H)2. 78(m, 2H) 4. 24(m, 2H)4. 52(m, 1H)4. 60(m 1H) 4. 98(m, 2H)6. 20(t, 1H)7. 41(t 2H) 7. 58(t, 2H)7. 94(s, 1H)8. 08(d, 2H) 9. 32(b, 1H) | 3282, 3085 1704, 1468, 1411, 1367 1319, 1276 1133, 688 |

The anti-cancer activities of the compounds of the present invention and their kinetics in blood were tested.

Pharmacological Test 1 (Anti-cancer Activity)

Method for Measuring Anti-tumor Activity

1) Formulation of Sample

The compound obtained in Examples 1, 4, 9 or 11 was uniformly suspended in 0.5% aqueous methylcellulose solution. The compound obtained in Example 14 was uniformly suspended in 0.5% aqueous methylcellulose solution containing 0.1% TWEEN 80.

2) Animals Used

For the compounds obtained in Examples 1, 4, 9 and 11, female $CDF_1$ mice of 7 weeks old were used. For the compound obtained in Example 14, female $CDF_1$ mice of 6 weeks old were used. Each group consisted of 5 or 6 animals.

3) Tumor Used

As the tumor, Meth A fibrosarcoma was used.

4) Testing Method

To the right abdomen of each mouse, 2 x $10^6$ tumor cells were subcutaneously transplanted. On the next day (day 1), the mice were randomly grouped and the suspension was compulsively administered to the mice by using an oral sound for successive 7 days (day 1 - day 7).

5) Evaluation of Anti-tumor Activity

On day 14, the tumors were removed and weighed. The tumor growth ratio (%) was calculated according to the following equation:

Tumor Growth Ratio (%) = T/C x 100

wherein T means the average weight of tumor of the treated group and C means the average weight of tumor of the control group. The results about the compounds obtained in Examples 1, 4, 9 and 11 are shown in Table 2, and the results about the compound obtained in Example 14 are shown in Table 3. In Table 2, "HCFU" means 1-hexyl carbamoyl-5-fluorouracil known as "Carmofur".

Table 2

| Test Compound (Example No.) | Dose (mg/kg) | Bodyweight Change (g) | Tumor Weight Ratio (T/C:%) |
|---|---|---|---|
| Example 1 | 14.0<br>56.1 | -0.4<br>-3.2 | 28.4<br>7.1 |
| Example 4 | 14.6<br>58.5 | -0.8<br>-3.8 | 23.3<br>4.8 |
| Example 9 | 15.0<br>60.0 | -0.9<br>-4.3 | 22.5<br>5.1 |
| Example 11 | 13.8<br>55.1 | -0.7<br>-4.4 | 27.6<br>5.8 |
| HCFU | 100.0 | -2.0 | 18.0 |
| Control | | 2.1 | 100.0 |

Table 3

| Test Compound (Example No.) | Dose (mg/kg) | Bodyweight Change (g) | Tumor Weight Ratio (T/C:%) |
|---|---|---|---|
| Example 14 | 13.6<br>54.4 | -0.5<br>-3.7 | 29.4<br>7.2 |
| Control | | 1.2 | 100.0 |

Pharmacological Test 2 (Kinetics in Blood)

1) Administration to Rats and Collection of Blood

The compounds according to the present invention prepared in Examples 4 and 9 were suspended in 0.5% carboxymethyl cellulose solution and the suspension was orally administered to rats by using an oral sound. After 1, 3, 6 or 9 hours from the administration, the rats were anesthetized with chloroform and 5 ml of blood was collected from abdominal inferior vena cava after opening their abdomina.

2) Extraction

The blood was centrifuged and plasma was collected. To 200 μl of the plasma, 10 μg/200 μl of 5-fluorouracil (used as an internal standard in HPLC) and 4 ml of ethyl acetate were added. The resulting mixture was shaken and then centrifuged. Then 3 ml of the ethyl acetate layer was placed in another test tube and evaporated to dryness under nitrogen gas blow. To the obtained residue, 200 μl of 50% acetonitrile/25 mM sodium phosphate (pH 4.0) was added and the resultant was subjected to ultrasonication. Then the insoluble materials were removed by centrifugation and the supernatant was collected.

3) Analysis by HPLC

Using a gel permeation column for analysis of nucleic acids (Asahipak GS-320H), 50 μl of the thus obtained supernatant was subjected to the analysis by HPLC. The flow rate was 1.0 ml/min. and the detection was carried out at 254 nm. As the mobile phase, 50% acetonitrile and 25 mM sodium phosphate (pH 4.0) were used. The quantification of F3Thd was carried out according to absolute calibration method using Chromatocorder 12.

The results are shown in Fig. 1. As shown in Fig. 1, the compounds of the present invention exist in blood in 1 - 6 hours after the administration and even at 9 hours after administration, they existed in a detectable level.

## Claims

**1.** A thioester derivative of 3'-O-propargyltrifluorothymidine, which is represented by the formula (1):

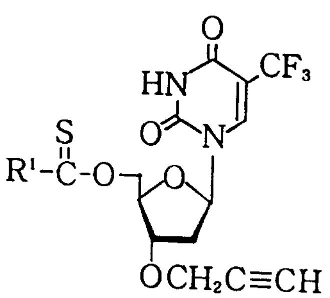

(1)

(wherein $R^1$ represents phenyl group; phenyl group substituted with 1 to 5 halogen atoms; phenyl group substituted with 1 to 5 $C_1$ - $C_4$ alkyl groups; phenyl group substituted with 1 to 5 $C_1$ - $C_4$ alkoxyl groups; phenyl group substituted with 1 to 5 $C_1$ - $C_4$ alkylthio groups; phenyl group substituted with 1

to 5 $C_1$ - $C_5$ alkanoylamino groups; phenyl group substituted with 1 to 5 trifluoromethyl groups; furyl group; thienyl group or pyrrolyl group).

**2.** The thioester derivative according to claim 1, wherein $R^1$ in the formula (1) is phenyl group.

**3.** The thioester derivative according to claim 1, wherein $R^1$ in the formula (1) represents phenyl group substituted with 1 to 5 halogen atoms; phenyl group substituted with 1 to 5 $C_1$ - $C_4$ alkyl groups; phenyl group substituted with 1 to 5 $C_1$ - $C_4$ alkoxyl groups; phenyl group substituted with 1 to 5 $C_1$ - $C_4$ alkylthio groups; phenyl group substituted with 1 to 5 $C_1$ - $C_5$ alkanoylamino groups; phenyl group substituted with 1 to 5 trifluoromethyl groups; furyl group; thienyl group or pyrrolyl group.

**4.** The thioester derivative according to claim 1, which is represented by the formula (2):

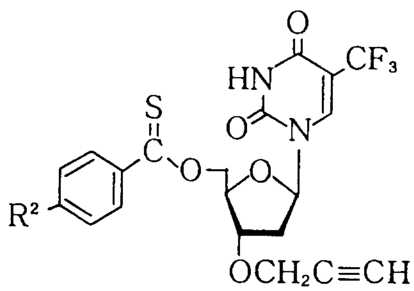

(2)

(wherein $R^2$ represents hydrogen atom, chlorine atom, methyl group or thiomethyl group).

**5.** A process for producing the thioester derivative according to claim 1, comprising reacting a 3'-O-propargyl-trifluorothymidine derivative of the formula (3):

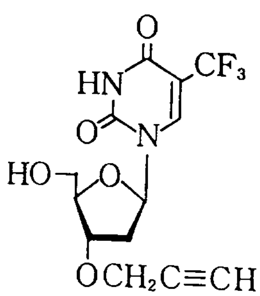

(3)

with a compound of the formula (4):

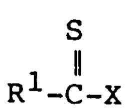

(4)

(wherein $R^1$ represents the same meaning as in formula (1), and X represents a halogen atom) in the presence of a base.

**6.** A process for producing the thioester derivative according to claim 1, comprising reacting a 3'-O-propargyl-trifluorothymidine derivative of the formula (3):

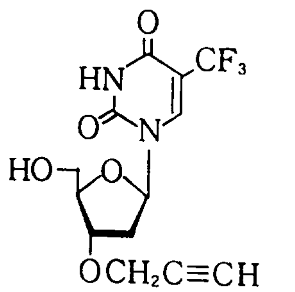

(3)

with a carboxymethyldithioester derivative of the formula (5):

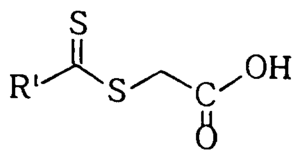

(5)

(wherein $R^1$ represents the same meanings as in formula (1))
in the presence of a base.

**7.** A pharmaceutical composition comprising the thioester derivative according to any of claims 1 to 4 as an effective ingredient.

**8.** The composition according to claim 7 comprising additionally a pharmaceutically acceptable carrier and/or diluent.

**9.** Use of the thioester derivative according to any of claims 1 to 4 for the preparation of a pharmaceutical composition useful as an anti-cancer agent.

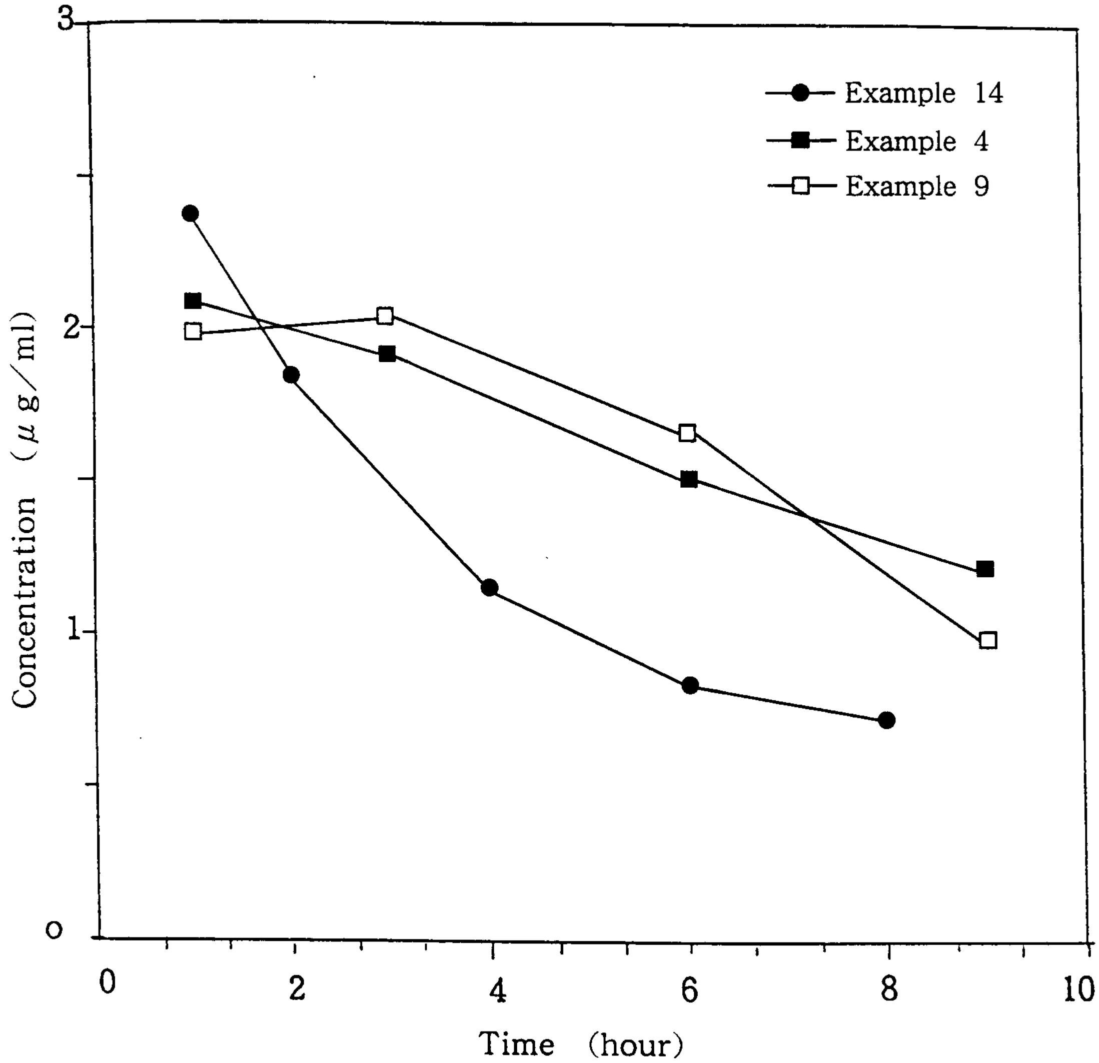
Example 14
Example 4
Example 9
3
2
1
o
0
2
4
6
8
10
Concentration (μg/ml)
Time (hour)

Fig. 1

European Patent Office

# EUROPEAN SEARCH REPORT

Application Number
EP 93 11 9638

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| P,X | EP-A-0 517 262 (MITSUI TOATSU CHAMICALS INC.)<br>* the whole document *<br>--- | 1-9 | C07H19/073<br>A61K31/70 |
| A | EP-A-0 129 984 (TAIHO PHARMACEUTICAL CO., LTD.)<br>* abstract *<br>--- | 1,7-9 | |
| A | CHEMICAL ABSTRACTS, vol. 112, no. 17, 23 April 1990, Columbus, Ohio, US;<br>abstract no. 158845v,<br>K.KOBAYASHI ET AL. 'Preparation of 5-iodo- or 5-(trifluoromethyl)-2'-deoxyuridine-5'-phosphates as Antitumour and Antiviral Agents.'<br>page 776 ;column 2 ;<br>* abstract *<br>& JP-A-01 261 396 (TAIHO PHARMACEUTICAL CO., LTD....) 18 October 1989<br>--- | 1,7-9 | |
| A | CHEMICAL ABSTRACTS, vol. 110, no. 3, 16 January 1989, Columbus, Ohio, US;<br>abstract no. 24248c,<br>T.UEDA ET AL 'Preparation of 5'-O-trityl-2'-deoxy-5-trifluoromethyluridine Derivatives as Intermediates for Antitumour Agents.'<br>page 589 ;column 1 ;<br>* abstract *<br>& JP-A-63 179 888 (TAIHO PHARMACEUTICAL CO., LTD....) 23 July 1988<br>---<br>-/-- | 1,7-9 | **TECHNICAL FIELDS SEARCHED (Int.Cl.5)**<br>C07H<br>A61K |

The present search report has been drawn up for all claims

| Place of search | Date of completion of the search | Examiner |
|---|---|---|
| THE HAGUE | 26 January 1994 | Scott, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 93 11 9638

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | CHEMICAL ABSTRACTS, vol. 112, no. 7, 12 February 1990, Columbus, Ohio, US; abstract no. 56520t, A.ROSOWSKY ET AL. 'Synthesis of 3'-O-propargylthymidine as a Candidate Antiretroviral Agent.' page 836 ;column 1 ; * abstract * & NUCLEOSIDES AND NUCLEOTIDES vol. 8, no. 4 , 1989 pages 491 - 497<br>----- | 1 | |

TECHNICAL FIELDS SEARCHED (Int.Cl.5)

The present search report has been drawn up for all claims

| Place of search | Date of completion of the search | Examiner |
|---|---|---|
| THE HAGUE | 26 January 1994 | Scott, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document